Europäisches Patentamt

**European Patent Office**

**Office européen des brevets**

(11) Publication number: **0 161 638 B1**

## EUROPEAN PATENT SPECIFICATION

(45) Date of publication of patent specification: **03.04.91**

(51) Int. Cl.⁵: **C12P 21/08**, C12N 5/02, C12N 15/00, G01N 33/577, G01N 33/53, //(C12P21/00, C12R1:91)

(21) Application number: **85105712.5**

(22) Date of filing: **09.05.85**

(54) Monoclonal antibody and method for quantitation of immoglobulins using the same.

(30) Priority: **11.05.84 JP 94117/84**

(43) Date of publication of application:
**21.11.85 Bulletin 85/47**

(45) Publication of the grant of the patent:
**03.04.91 Bulletin 91/14**

(84) Designated Contracting States:
**AT BE CH DE FR GB IT LI LU NL SE**

(56) References cited:
**EP-A- 0 044 219**

**CLINICAL CHEMISTRY, vol. 27, no. 12, 1981, pages 2044-2047; I. DEVERILL et al.: "Monoclonal antibodies to human IgG: Reaction characteristics in the centrifugal analyzer"**

(73) Proprietor: **WAKO PURE CHEMICAL INDUSTRIES, LTD.**
**10, Doshomachi-3-chome**
**Higashi-ku Osaka(JP)**

(72) Inventor: **Miyashita, Yoshinobu**
**7-14-17 Takadono**
**Asahi-ku Osaka(JP)**
Inventor: **Tsubaki, Kazuyuki**
**19-69 Aza Shireta**
**Yamada Itami-shi(JP)**
Inventor: **Kobatake, Shinzo**
**A10-206, 18 Yamadanishi-2-chome**
**Suita-shi(JP)**
Inventor: **Ushio, Yoshihiro**
**11-3-307 Kitanatsugicho**
**Nishinomiya-shi(JP)**
Inventor: **Suzuoki, Jun**
**14-1 Okamoto-7-chome**
**Higashinada-ku Kobe(JP)**

Rank Xerox (UK) Business Services

CHEMICAL ABSTRACTS, vol. 94, no. 11, 16th March 1981, page 535, abstract no. 81866c, Columbus, Ohio, US; J. STEENSGAARD et al.: "The development of difference turbidimetric analysis for monoclonal antibodies to human IgG"

EUR. J. IMMUNOLOGY, vol. 6, 1976, pages 292-295; G. KÖHLER et al.: "Fusion between immunoglobulin-secreting and nonsecreting myeloma cell lines"

(74) Representative: **Baillie, Iain Cameron et al c/o Ladas & Parry Isartorplatz 5 W-8000 München 2(DE)**

**Description**

BACKGROUND OF THE INVENTION

This invention relates to an optical method for the assay of human immunoglobulins by using monoclonal antibodies thereto either alone or in mixtures of two or more antibodies.

Immunoglobulins are proteins produced in lymphoid cells particularly plasma cells and play an important role as antibodies in protecting living bodies from bacteria, viruses, and toxins. The assay of immunoglobulin content of body fluids is an important means for the diagnosis, prophylaxis, and prognosis of various diseases.

In recent years, remarkable progress has been made in the method of assay for the immunoglobulin in body fluids; recently developed assay methods include SRID, laser nephelometry, turbidimetry, RIA, EIA, and latex agglutination. Especially, a method of rapid assay for immunoglobulin by means of a laser nephelometer as an autoanalyzer for the biochemical assay of multiple items has been lately developed and becoming now in wide use.

However, the antibody used in the above methods is generally a polyclonal one and, as a consequence, the degree of agglutination caused by the antigen-antibody reaction is so high that it is necessary to dilute the sample serum before being assayed. Such dilution makes the assay procedure complicated and tends to introduce an error into the results of assay.

Furthermore, the assay by an automatic analyzer is performed, as a rule, on an undiluted sample. As a consequence, hitherto it has been practically impossible to assay the immunoglobulin content simultaneously with biochemical examination of sugars, lipids, enzymes, and the like on the same sample. Of the immunoglobulins in the serum, IgG is present in an extremely high concentration, the ordinary content being 500 to 3,000 mg/dl. Accordingly, in an optical method of assay such as turbidimetry or nephelometry using polyclonal antibodies derived from an immunized animal, if the sample is an undiluted serum, a large amount of the antibodies becomes necessary, resulting in an extremely high turbidity which makes the optical measurement impossible. Therefore, previously it was generally believed impossible to carry out the measurement on an undiluted sample. It was necessary for the optical measurement to carry out the antigen-antibody reaction in a serum sample of 10 to 30 fold dilution or, in some cases, even 100 to 300 fold dilution (e.g. nephelometry).

On the other hand, as examples of the methods of clinical chemical analysis using monoclonal antibodies in the antigen-antibody reaction, there may be mentioned radioimmunoassay (RIA), enzyme immunoassay (EIA), and fluoroimmunoassay (FIA). In these methods, the antigen or antibody being assayed is labeled with an isotope, enzyme, or fluoroscent substance and after completion of the antigen-antibody reaction the amount of antigen or antibody is indirectly determined through the measurement of the substance used in the labeling, the monoclonal antibody being used in order to improve the precision of assay. However, no example of the effective use of monoclonal antibodies is found in the case of direct optical measurement of the degree of agglutination resulting from the antigen-antibody reaction. A reason for this seems to be as described below.

In the case wherein the antigen is an immunoglobulin such as IgG having only two antigenic determinants per molecule, the IgG molecule can combine with a monoclonal antibody molecule only at two sites and the resulting complex sill not sufficiently grow in particle size to increase the turbidity, contrary to the case of polyclonal antibodies originated from an animal, wherein many antibody molecules, not predeterminable in number, combine with one IgG molecule to increase the particle size of antigen-antibody complex, resulting in an increase in turbidity. Therefore, it has been generally believed that with monoclonal antibodies it is impossible to increase the particle size of antigen-antibody complex and, hence, the turbidity per unit weight of the antibody to a sufficiently high level suitable for the optical measurement.

As is well known to the art, there is a report on the method of assay for immunoglobulin by use of monoclonal antibodies in Clinical Chemistry, Vol. 27, pp. 2044-2047 (1981). The report may be summarized as follows: Although there is observed no turbidity due to the antigen-antibody reaction when a single monoclonal antibody is used, yet a turbidity sufficient for the measurement is obtained by use of combinations of two or more monoclonal antibodies. Therefore, when used alone, the monoclonal antibodies reported in the above literature produce, upon reaction with antigens, no or scarcely perceptible turbidity. To produce a turbidity comparable to that produced by polyclonal antibodies, it is necessary to use a number of monoclonal antibodies; such a combination can replace polyclonal antibodies. In this case, of course the sample should be diluted similarly to the case wherein the conventional polyclonal antibodies are used.

EP 0 161 638 B1

## SUMMARY OF THE INVENTION

The present invention provides a method for the assay of immunoglobulin, which comprises using monoclonal antibodies to human immunoglobulin, which, when used singly or in mixtures, are capable of producing turbidity upon reacting with an antigen, said monoclonal antibodies being produced by a hybridoma formed by the fusion between cells from mouse myeloma cell line and spleen cells derived from the mouse immunized with human immunoglobulin; allowing said monoclonal antibodies and an undiluted sample of body fluids to undergo the antigen-antibody reaction; exposing the resulting antigen-antibody complex to light; and optically measuring the degree of agglutination caused by the antigen-antibody reaction.

This invention makes it possible to assay immunoglobulins in body fluids on a biological sample such as serum, as such without sample dilution, by optically measuring the degree of agglutination caused by the antigen-antibody reaction. As a consequence, precision of the assay is improved by eliminating the complication of procedure and the error associated with dilution of samples. It is also made possible to incorporate the assay of immunoglobulins into multi-item measurements on a single sample, carried out by an automatic analyzer. Therefore, this invention is expected to contribute much to the art.

## BRIEF DESCRIPTION OF THE DRAWINGS

Fig. 1 is a graph representing the relationship between the quantity of IgG and the absorbance (OD).
Fig. 2 is a graph representing the relationship between the quantity of IgM and the absorbance (OD).
Fig. 3 is a graph representing the relationship between the quantity of IgG and the light scattering intensity.

## DESCRIPTION OF THE PREFERRED EMBODIMENTS

The present inventors made an extensive study to find an optical method of assaying or determining quantitatively human immunoglobulin in an undiluted sample. As a result, it was found that monoclonal antibodies to human immunoglobulin, when used singly (that is, not a combination of a number of monoclonal antibodies), produce turbidity upon reaction with an antigen and found that by using said monoclonal antibodies, it is possible to assay optically the human immunoglobulin content of an undiluted sample of body fluids such as serum; the sensitivity (i.e. the degree of turbidity) can be suitably adjusted by using said monoclonal antibodies each alone or in mixtures of two or more of them. The present invention is predicated upon the above discovery.

The assay using the monoclonal antibodies of this invention is carried out in the concentration range somewhat lower than that when polyclonal antibodies are used. In fact, however, it was found that the concentration range is favorable for the assay of immunoglobulin in an undiluted sample of body fluids such as serum, the turbidity (sensitivity) being sufficient for the optical measurement. The assay on an undiluted sample results in an improvement of the precision because of the elimination of complicated procedure and the error of measurement associated with dilution. Moreover, it makes possible to include the immunoglobulin assay into multi-item biochemical examination for sugars, lipids, enzymes, and so on, carried out on a single sample by means of an automatic analyzer, resulting in an extension of the field of application of the present method.

The first stage is the formation of monoclonal hybridoma excreting the antibodies, which comprises essentially the following three steps:

1. Immunization.
2. Cell fusion.
3. Selection and cloning of the hybridoma. The procedures in each step are described below in the order indicated.

The antigens used in the immunization are purified immunoglobulins separated from the human serum. Each immunoglobulin is dissolved in physiological saline or buffer and injected to mouse or rat preferably at a dose of 1 to 300 μg. The immunization is performed several times at predetermined intervals. The primary immunization is generally performed together with an adjuvant such as Freund's adjuvant or alum. The administration is repeated at intervals of 2 to 4 weeks. The final immunization is performed by administrating intraperitoneally or intravenously a solution of an immunoglobulin in physiological saline without using an adjuvant. The animal being immunized is usually rat or mouse, the selection of strain being dependent upon the established tumor cell strain used in the cell fusion. Of the mouse strains, the most

4

frequently used is BALB/C which is related to an established tumor cell strain producing no immunoglobulin. Two to four days after the final immunization, the lymph node or the spleen is excised from the animal body and the lymphocyte obtained therefrom is used in the cell fusion.

The tumor cell strain used in the cell fusion is P3-X63-8AZ-1 or P3-NS1-1 which derived from P3-X63 mouse myeloma cell line producing no immunoglobulin. In the cell fusion, the lymphocyte is used in an amount of 5 to 20 times the tumor cells. After washing with MEM medium, McCoy medium, RPMI 1640 medium, or an isotonic buffer, the lymphocyte and the tumor cell are centrifugally treated to the pellet form. The loosened pellets are subjected to cell fusion in the presence of Sendai virus (HVJ) or polyethylene glycol (PEG). It is convenient for handling to use 0.5 to 2 ml of a 40 to 60% solution of PEG having an average molecular weight of 1,000 to 8,000. A small amount of dimethyl sulfoxide is sometimes added together with PEG to accelerate the fusion but it is not essential. After the addition of PEG, the cell fusion is allowed to proceed for 1 to 10 minutes. To the reaction mixture is then added slowly 10 to 50 ml of MEM or RPMI 1640 medium to terminate the reaction. Immediately after termination of the cell fusion, the mixture is centrifuged to remove the supernatant. The fused cells are suspended in MEM or RPMI 1640 medium containing 5 to 20% of fetal calf serum (FCS). The suspension is fed to a 24-well culture plate in an amount of 1 ml per well so that $1 \times 10^5$ to $5 \times 10^6$ lymphocytes may be present in each well. It is desirable to add feeder cells such as thymus cells or spleen cells of rat or mouse of the same line, the concentration of the feeder cells in the medium being $(0.5-2) \times 10^6$/ml. The culture is replaced stepwise by RPM I 1640 medium (or MEM-medium) containing $1 \times 10^{-4}$ M hypoxanthine, $4 \times 10^{-7}$ M aminopterin, and $1.6 \times 10^{-5}$ M thymidine, that is HAT medium. The replacement is generally carried out in the following manner: On the day succeeding the day of feeding the suspension to each well of the culture plate, the same volume of HAT medium as that fed to each well is added to each well. On the next day, half volume of the medium in each well is replaced by the equal volume of HAT medium. Then, every 3 or 4 days half volume of the medium is replaced by the equal volume of HAT medium. On the 10-14th day after fusion, half volume of the medium is replaced by the equal volume of HT medium (HAT medium minus aminopterin). Then, every 1-3 days half valume of the medium is replaced by the equal volume of ordinary medium containing no HAT. The supernatant of the culture medium in the wells where the growth of fused cells (hybridoma) is actively in progress is examined by various analyzing techniques such as, for example, RIA and ELISA to select the hybridoma producing the intended antibody. The selected hybridoma is then cloned by means of a fluorescent activated cell sorter (FACS) or by other methods such as method of picking up a colony from soft agar or method of limiting dilution which is widely used. The cloning is repeated twice or more to secure a perfect monoclone. Once monoclones have been established, the cells of each colone are cultured in vitro or in vivo to produce various monoclonal antibodies to respective human immunoglobulins. The culture in vivo is preferred because of a much higher antibody titer.

The assay of immunoglobulins by use of the monoclonal antibodies according to this invention is suitably performed by turbidimetry, nephelometry, or the like. The turbidimetric assay by use of the present monoclonal antibodies can be carried out by means of a known automatic analyzer or a spectrophotometer, the preferred range of wave length being from 340 to 800 nm, though not limitative. In the nephelometric assay a nephelometer with laser as a light source can be used but the invention is not limited to any particular light source or the method of detection.

The buffers used in the assay include those which are generally used, such as Tris-buffer, phosphate buffer, and veronal buffer. The pH is preferably in the range of 6.0 to 9.0, though not limitative.

The type and number of monoclonal antibodies used in the assay are not limited. Depending upon the required sensitivity, the monoclonal antibodies can be used each alone or in mixtures of two or more of them. The amount used of the monoclonal antibody is not limited but generally 0.02 to 2 g of a monoclonal antibody is present in 1 ml of the antibody solution.

The immunoglobulins which can be assayed by the method of this invention include human immunoglobulin classes IgG, IgA, and IgM.

The invention is illustrated below in further detail with reference to Examples, but the invention is not limited thereto.

## EXAMPLE 1

Preparation of monoclonal anti-human IgG antibody and hybridoma producing same.

1. Immunization.

An immunogen was prepared by mixing 100 μg of human IgG in 0.1 ml of 0.15 M sodium chloride solution and 0.1 ml of complete Freund's adjuvant. A 0.2 ml portion of the emulsion was intraperitoneally administered to a BALB/C mouse (female, 6 weeks of age). After 4 weeks, 100 $\mu$g of human IgG in 0.2 ml of 0.15 M sodium chloride solution was injected into the tail vein of the mouse.

2. Cell fusion.

Three days after the final immunization, the spleen was removed from the immunized mouse. The spleen is teased in a plastic Petri dish containing 10 ml of RPMI 1640 medium and a cell suspension was made by filtering through a stainless steel mesh. After washing three times with RPMI 1640 medium by repeated centrifugation (1000 rpm, 10 minutes), cell fusion was carried out. $1 \times 10^8$ spleen splenocytes and $1 \times 10^7$ mouse myeloma cells P3-NS1-1 were fused in 1 ml of 50% polyethylene glycol 6000 (PEG6000). Cell fusion was allowed to take place for 1 minutes at room temperature, while rotating the test tube. Every 30 seconds, 1 ml of RPMI 1640 medium was added to the test tube over a period of 5 minutes to stop the fusion reaction. The mixture was immediately centrifuged (1,000 rpm, 5 minutes) to remove the supernatant and the fused cells were suspended in 50 ml of RPMI 1640 medium containing $1 \times 10^6$/ml of feeder cells and 20% of fetal calf serum (FCS). The cell suspension was seeded into two 24-wells culture plates so that each well may contain 1 ml of the suspension and the culture plates were incubated in a $CO_2$-incubator.

After 24 hours, to each well was added 1 ml of HAT medium (RPMI 1640 medium admixed with 20% FCS containing $1 \times 10^{-4}$ M hypoxanthine, $4 \times 10^{-7}$ M aminopterin, and $1.6 \times 10^{-5}$ M thymidine). On the second day, third day, and thereafter every two days the half volume of the culture medium in each well was replaced by equal volume of HAT medium. On the tenth day, the half volume of the culture medium in each well was replaced by equal volume of HT medium (HAT medium minus aminopterin). From the next day on, every two days the half volume of the culture medium in each well was replaced by equal volume of normal medium (RPMI 1640 medium plus 10% FCS). The culture supernatant collected on 18th day was examined for anti-human IgG antibody production.

3. Selection of hybridoma.

For the purpose of selecting hybridoma capable of producing anti-human IgG antibody, each culture supernatant in 48 wells was assayed by ELISA. For this purpose, each well of the 96-well culture plate for ELISA was fed with 0.1 ml of human IgG of 10 $\mu$g/ml in concentration and left standing at 4°C for 16 hours to fix the human IgG to the culture plate. Each well was washed three times with a washing solution [10 mM phosphate buffer of pH 7.4 and containing 0.05% of Tween 20 (Registered Trade Mark) (a nonionic surface active agent of Atlas Co.)]. Each well was fed with 0.2 ml of 1% bovine serum albumin solution in order to avoid nonspecific adsorption of the proteins in the culture supernatant. The culture plate was left standing at 37°C for 2 hours. After washing three times with the washing solution, each well was fed with 0.1 ml of the cell culture supernatant and left standing at 37°C for 2 hours.

For a negative reference, 0.1 ml of RPMI 1640 medium with the addition of 20% FCS was fed. After individual wells were washed three times with the washing solution, then fed with 0.1 ml of a solution of anti-mouse-immunoglobulin antibody labeled with peroxydase, and left standing at 37°C for 2 hours. After washing three times with the washing solution, 0.1 ml of a 0.4% 0-phenylenediamine solution was added and alowed to react for 2 minutes at room temperature. The reaction was stopped by adding 0.05 ml of 6 N sulfuric acid and absorbance at 490 nm was measured. The hybridoma growing in a culture supernatant which showed an absorbance twice or more as large as that of the negative reference was selected as the hybridoma capable of producing an anti-human IgG antibody. Among 48 wells tested, three showed the production of anti-human IgG antibody.

4. Cloning.

In order to prepare feeder cells, the thymus was removed from a BALB/C mouse (female, 6 weeks old) and was teased in a plastic Petri dish containing 10 ml of RPMI 1640 medium. The thymocytes were washed three times with RPMI 1640 medium by repeated centrifugation (1000 rpm for 10 minutes) and suspended in 50 ml of RPMI 1640 medium with the addition of 20% FCS. To the feeder cells suspension, was added 0.1 ml of a hybridoma solution containing 500 cells/ml capable of producing anti-human IgG antibodies. After thorough mixing, 0.2 ml of the mixture was seeded into a 96-wells culture plate and incubated for 10 days in a $CO_2$-incubator. The culture supernatants, in which the growth of cells was noticed, were assayed by ELISA to sort out 7 clones of hybridoma capable of producing anti-human IgG antibodies. Among these clones, one clone was again subjected to the second cloning to yield 6 clones of hybridoma capable of producing anti-human IgG antibodies.

5. Preparation of monoclonal antibodies.

$2 \times 10^6$ cells of the clone G-2-3 obtained by the steps from 1 to 4 through descrived above were suspended in 0.2 ml of RPMI 1640 medium and the suspension was intraperitoneally administered to BALB/C mouse primed with pristan (male, 6 weeks of age) to collect ascites. To 4 ml of the ascites, was added gradually a saturated ammonium sulfate solution until the final ammonium sulfate concentration had reached 50%. The mixture was stirred at room temperature for 2 hours and the precipitate was collected by centrifuging (3,000 rpm, 10 minutes). The precipitate was dissolved in 5 ml of physiological saline. The serial 10-fold dilutions of the reconstituted solution were assayed for the antibody activity by ELISA. The maximum dilution of detectable antibody activity was $10^6$. The mouse IgG content was found to be 23 mg/ml, as tested with an agarose plate containing rabbit anti-mouse IgG antibody (SRID method).

6. Two other clone G3 and G5 were also obtained by the similar procedures described for G-2-3 in steps from theory 5. The antibody activity and the mouse IgG contents were measured to obtain $10^6$ and 22 mg/ml for G3 and $10^5$ and 20 mg/ml for G5 respectively.

EXAMPLE 2

Preparation of monoclonal anti-human IgG antibody and hybridoma producing same.

An immunogen was prepared by mixing 200 $\mu$g of human IgA in 0.1 ml of 0.15 M sodium chloride solution and 0.1 ml of complete Freund's adjuvant. A 0.2 ml portion of the emulsion was intraperitoneally administered to BALB/C mouse (male, 5 weeks old). After 4 weeks, 100 $\mu$g of human IgA in 0.2 ml of 0.15 M sodium chloride solution was injected into the tail vein. In a manner similar to that described in steps 2 to 4 of Example 1, 5 clones of hybridoma capable of producing anti-human IgA antibodies were obtained. In a manner similar to that in step 5 of Example 1, 5 monoclonal anti-human IgA antibodies A-2, A-3, A-5, A-8, and A-9 were obtained. The antibody activities and the mouse IgG contents were as shown in Table 1.

## Table 1

| Anti-human IgA clone No. | Antibody activity | Mouse IgG content (mg/ml) |
|---|---|---|
| A - 2 | $10^5$ | 18 |
| A - 3 | $10^6$ | 24 |
| A - 5 | $10^6$ | 21 |
| A - 8 | $10^5$ | 22 |
| A - 9 | $10^6$ | 25 |

EXAMPLE 3

Preparation of monoclonal anti-human IgM antibody and hybridoma producing same.

An immunogen was prepared by mixing 100 $\mu$g of human IgM in 0.1 ml of 0.15 M sodium chloride solution and 0.1 ml of complete Freund's adjuvant. A 0.2 ml portion of the emulsion was intraperitoneally administered to BALB/c mouse (female, 6 weeks old). After 3 weeks, 100 $\mu$g of human IgM in 0.1 ml of 0.15 M sodium chloride solution was injected into the tail vein. In a manner similar to that described in steps 2 to 4 of Example 1, 4 clones of hybridoma capable of producing anti-human IgM antibody were obtained. In a manner similar to that in step 5 of Example 1, 4 monoclonal antibodies M-1, M-2, M-3, and M-5 were obtained. The antibody activities and the mouse IgG contents were as shown in Table 2.

7

EP 0 161 638 B1

## Table 2

| Anti-human IgM clone No. | Antibody activity | Mouse IgG content (mg/ml) |
|---|---|---|
| M - 1 | $10^6$ | 23 |
| M - 2 | $10^6$ | 22 |
| M - 3 | $10^5$ | 24 |
| M - 5 | $10^6$ | 20 |

EXAMPLE 4

Estimation of human serum IgG by using monoclonal antibody.

### 1. Buffer:

| | |
|---|---|
| Polethylene glycol 6000 | 5 g |
| 0.1 M TRIS buffer (pH 7.4) | 100 ml |

### 2. Antibody solution:

| | |
|---|---|
| Monoclonal antibody G-3 obtained in Example 1 | 1 ml |
| Buffer | 50 ml |

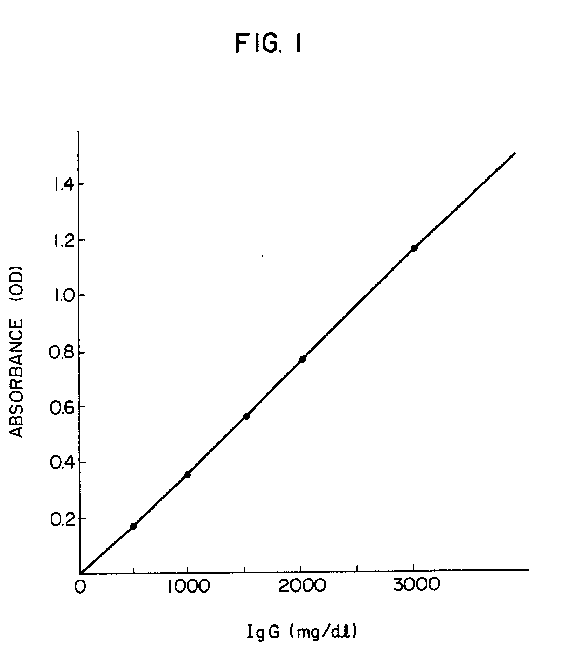

Samle: The human sera in which IgG content of 500, 1,000, 1,500, 2,000, and 3,000 mg/dl were used.
Procedure: Each 10 $\mu$l of the five samples was placed in a test tube. To each test tube was added 2 ml of the antibody solution. Each reaction mixture was allowed to react at 37° C for 10 minutes. The absorbance of reaction mixture was measured at 505 nm by a HITACHI 624 spectrophotometer under the conditions: effective cell lentgh of 10 mm.
The results of measurement were as shown in Fig. 1.

EXAMPLE 5

Estimation of human serum IgA using monoclonal antibody.
Reagent: The following reagents were prepared.

8

## 1. Buffer:

| | |
|---|---|
| Polyethylene glycol 6,000 | 4.5 g |
| Sodium chloride | 0.9 g |
| 0.01 M phosphate buffer (pH 7.0) | 100 ml |

## 2. Antibody solution:

| | |
|---|---|
| Monoclonal antibody A-2 obtained in Example 2. | 0.2 ml |
| Monoclonal antibody A-3 in Example 2 | 0.2 ml |
| Buffer | 100 ml |

## 3. Standard serum:

Standard serum in which IgA content was 560 mg/dl was used.

Samples: Human sera

Procedures: Each 10 $\mu\ell$ of standard serum and human sera as such was placed in a test tube. After addition of 3 ml of the antibody solution or the buffer solution, the mixture was allowed to react at 37° C for 10 minutes. The absorbance of the reaction mixture was measured at 375 nm by a HITACHI 624 spectrophotometer. The path length in the cuvette was 10 mm.

Calculation:

$$\text{IgA content in human serum (mg/dl)} = \frac{E_s - (E_B + E_{s \cdot B})}{E_{std} - (E_B + E_{std \cdot B})} \times 560$$

where

$E_s$: Absorbance of reaction mixture of human serum and antibody solution

$E_{s\,B}$: Absorbance of mixture of human serum and buffer.

$E_{std}$: Absorbance of reaction mixture of standard serum and antibody solution.

$E_{std\,B}$: Absorbance of mixture of standard serum and buffer.

$E_B$: Absorbance of antibody solution.

The IgA content of human sera obtained by the present method and that quantitated by the SRID method were shown in Table 3.

EP 0 161 638 B1

## Table 3

| Human serum No. | Method of this invention, y (mg/dl) | Method of SRID, x (mg/dl) |
|---|---|---|
| 1 | 208 | 205 |
| 2 | 271 | 274 |
| 3 | 430 | 440 |
| 4 | 368 | 360 |
| 5 | 174 | 170 |
| 6 | 255 | 250 |
| 7 | 86 | 90 |
| 8 | 139 | 144 |
| 9 | 244 | 250 |
| 10 | 315 | 320 |

$$\gamma = 0.998$$

$$y = 0.99x + 0.53$$

As is shown in Table 3, the results obtained by the present method and those obtained by the SRID method correlate well.

EXAMPLE 6

Estimation of human sera IgM using monoclonal antibody.
Reagent: The following reagents were prepared.

**1. Buffer:**

| | |
|---|---|
| Polyethylene glycol 6,000 | 4 g |
| Sodium chloride | 0.9 g |
| 0.01 M veronal buffer (pH 7.5) | 100 ml |

**2. Antibody solution:**

| | |
|---|---|
| Monoclonal antibody M-1 obtained in Example 3 | 0.1 ml |
| Monoclonal antibody M-3 obtained in Example 3 | 0.1 ml |
| Monoclonal antibody M-5 obtained in Example 3 | 0.1 ml |
| Buffer | 100 ml |

Samples: The human sera in which IgM contents were 75, 150, 300, 500 and 700 mg/dl were used.

Procedures: Each 10 $\mu\ell$ of human sera was placed in a test tube. After addition of 2 ml of the antibody solution or the buffer solution, the mixture was allowed to react at 37°C for 10 minutes. The absorbance of the reaction mixture was measured at wavelength of 505 nm by a spectrophotometer (Type UV-200 of Shimadzu Seisakusho, Ltd.), the path length in the cuvette being 10 mm.

Calculation: Absorbance of reaction mixture of the serum and the antibody: $E_s = E_{s\,A} - E_{s\,B}$

where

$E_{s\,A}$: Absorbance of reaction mixture of human serum and antibody solution.

$E_{s\,B}$: Absorbance of a mixture of human serum and the buffer solution.

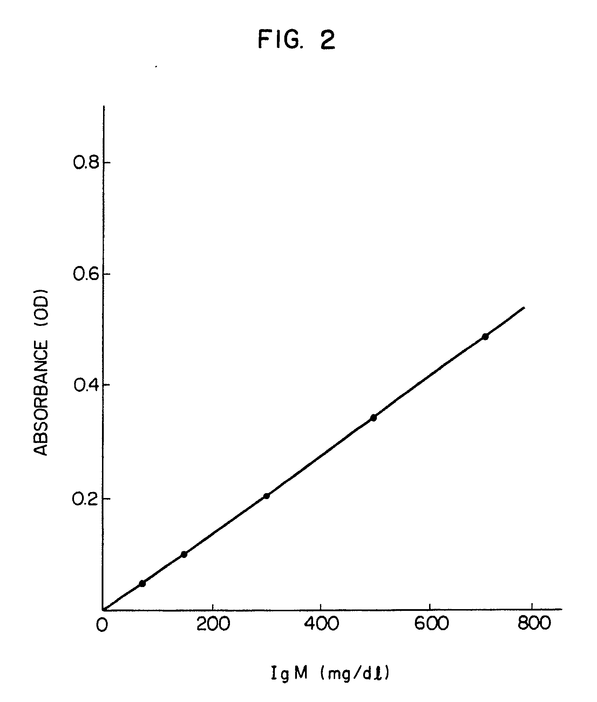

The results of measurement were as shown in Fig. 2.

EXAMPLE 7

Estimation of human serum IgG using monoclonal antibody.

Reagent: The following reagents were prepared.

## 1. Buffer:

| | |
|---|---|
| Polyethylene glycol 6,000 | 5 g |
| Sodium chloride | 0.9 g |
| 0.1 M TRIS buffer (pH 7.5) | 100 ml |

## 2. Antibody solution:

| | |
|---|---|
| Monoclonal antibody G-2-3 obtained in Example 1 | 0.5 ml |
| Monoclonal antibody G-5 obtained in Example 1 | 0.5 ml |
| Buffer | 50 ml |

## 3. Standard serum:

Standard sera in which the IgG contents were 500, 1,000, 2,000, and 4,000 mg/dl were used.

Sample: Human sera.

Precedures: Into a cuvette, was placed 10 $\mu\ell$ of the standard serum or a human serum without dilution, and then 500 $\mu\ell$ of the antibody solution or the buffer solution was added. The mixture was allowed to react at room temperature for 15 minutes and the scattered light intensity at a wavelength of 633 nm was measured by means of a laser nephelometer (ZD-801 of Wako Co.). The intensity of scattered light ($D_s$) after the human IgG and the antibody had reacted was calculated by the following equation

$$D_s = D_{s\,A} - D_{s\,B}$$

where
$D_{s\,A}$: Scattered light intensity [mV] of the reaction mixture of serum and antibody solution,
$D_{s\,B}$: Scattered light intensity (mV) of the mixture of serum and buffer.

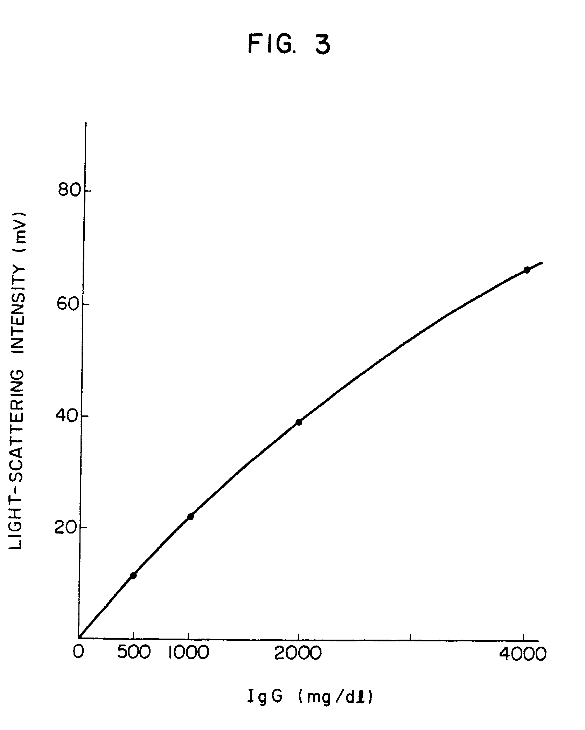

The standard curve was shown in Fig. 3. The IgG content of human serum samples was determined according to the standard curve. The results and that determined by the SRID method were shown in Table 4.

Table 4

| Human serum No. | Method of this invention, y (mg/dl) | Method of SRID, x (mg/dl) |
|---|---|---|
| 1 | 1135 | 1130 |
| 2 | 1404 | 1400 |
| 3 | 881 | 870 |
| 4 | 1627 | 1630 |
| 5 | 1565 | 1550 |
| 6 | 1312 | 1320 |
| 7 | 2760 | 2700 |
| 8 | 2250 | 2270 |
| 9 | 1500 | 1540 |
| 10 | 944 | 930 |
| 11 | 1871 | 1900 |
| 12 | 1356 | 1370 |

$$\gamma = 0.999$$

$$y = 1.01x - 15.48$$

As shown in Table 4, the results obtained by the present method and those obtained by the method of SRID correlate satisfactorily.

Claims

1. A method for the assay of immunoglobulins, which comprises using monoclonal antibody to human immunoglobulins, which, when used singly is capable of producing turbidity upon reacting with an antigen, said monoclonal antibody being produced by a hybridoma formed by the fusion between cells from a mouse myeloma line and spleen cells derived from the mouse immunized with human immunoglobulin, allowing said monoclonal antibody and an undiluted sample of body fluids to undergo the antigen-antibody reaction; exposing the resulting antigen-antibody complex to light, and optically measuring the degree of agglutination caused by the antigen-antibody reaction.

2. A method for the assay of immunoglobulin according to Claim 1, wherein the cells derived from a mouse myeloma cell line belong to P3-X63.

3. A method for the assay of immunoglobulin according to Claim 2, wherein the cells belonging to P3-X63 derived from a mouse myeloma cell line are P3-NS1-1.

13

EP 0 161 638 B1

4. A method for the assay of immunoglobulin according to any preceding claim, wherein the light-scattering intensity of the complex is measured.

5. A method for the assay of immunoglobulin according to any of Claims 1 to 3, wherein the difference in transmittance of the complex is measured.

6. A method for the assay of immunoglobulin according to any preceding claim, wherein the immunoglobulin is human IgG, human IgM or human IgA.

7. A method for the assay of immunoglobulin according to any preceding claim which comprises using mixtures of two or more of said monoclonal antibodies.

## Revendications

1. Procédé de dosage des immunoglobines qui comprend l'utilisation d'un anticorps monoclonal pour les immunoglobines humaines lequel, lorsqu'il est utilisé isolément, est capable de produire un trouble par réaction avec un antigène, cet anticorps monoclonal étant produit par un hybridome formé par la fusion entre des cellules d'une lignée de myélome de souris et des cellules de rats provenant d'une souris immunisée avec de l'immunoglobuline humaine, le fait de laisser cet anticorps monoclonal et un échantillon non dilué de fluides corporels subir la réaction antigène-anticorps ; l'exposition du complexe antigène-anticorps obtenu à la lumière, et le fait de mesurer optiquement le degré d'agglutination causé par la réaction antigène-anticorps.

2. Procédé de dosage de l'immunoglobine selon la revendication 1, dans lequel les cellules provenant d'une lignée cellulaire de myélome de souris appartiennent à P3-X63.

3. Procédé de dosage d'une immunoglobine selon la revendication 2, dans lequel les cellules appartenant à P3-X63 provenant d'une lignée cellulaire de myélome de souris sont des P3-NS1-1.

4. Procédé de dosage de l'immunoglobine selon l'une quelconque des revendications précédentes, dans lequel on mesure l'intensité de dispersion de la lumière du complexe.

5. Procédé pour le dosage de l'immunoglobine selon l'une quelconque des revendications 1 à 3, dans lequel on mesure la différence de transmittance du complexe.

6. Procédé pour le dosage de l'immunoglobine selon l'une quelconque des revendications précédentes, dans lequel l'immunoglobine est l'IgG humaine, l'IgM humaine ou l'IgA humaine.

7. Procédé de dosage de l'immunoglobine selon l'une quelconque des revendications précédentes, qui comprend l'utilisation de mélanges de plusieurs de ces anticorps monoclonaux.

## Ansprüche

1. Verfahren zum Bestimmen von Immunoglobulin, in dem ein monoklonaler Antikörper gegen Humanimmunoglobuline verwendet wird, der bei seiner alleinigen Verwendung zum Hervorrufen einer Trübung bei seiner Reaktion mit einem Antigen befähigt ist, wobei der Antikörper von einem Hybridom erzeugt wird, das durch die Verschmelzung von Zellen einer Zeile von Mausmyelomzellen und von mit Humanimmunoglobulin immunisierten Mäusen gewonnenen Milzzellen erzeugt worden ist, dem monoklonalen Antikörper und einer unverdünnten Probe von Körperflüssigkeiten die Durchführung der Antigen-Antikörper-Reaktion gestattet wird, der so erhaltene Antigen-Antikörper-Komplex belichtet wird und der Grad der durch die Antigen-Antikörper-Reaktion bewirkten Agglutination optisch gemessen wird.

2. Verfahren zur Analyse von Immunoglobulin nach Anspruch 1, dadurch gekennzeichnet, daß die von einer Mausmyelomzellenzeile abgeleiteten Zellen zum Typ P3-X63 gehören.

14

3. Verfahren zum Bestimmen von Immunoglobulin nach Anspruch 2, dadurch gekennzeichnet, daß die von einer Mausmyelomzellenzeile abgeleiteten Zellen vom Typ P3-X63 zum Typ P3-NS1-1 gehören.

4. Verfahren zum Bestimmen von Immunoglobulin nach einem der vorhergehenden Ansprüche, dadurch gekennzeichnet, daß die Lichtstreuintensität des Komplexes gemessen wird.

5. Verfahren zum Bestimmen von Immunoglobulin nach einem der Ansprüche 1 bis 3, dadurch gekennzeichnet, daß die Differenz der Lichtdurchlässigkeit des Komplexes gemessen wird.

6. Verfahren zum Bestimmen von Immunoglobulin nach einem der vorhergehenden Ansprüche, dadurch gekennzeichnet, daß das Immunoglobulin Human-IgG, Human-IgM oder Human-IgA ist.

7. Verfahren zum Bestimmen von Immunoglobulin nach einem der vorhergehenden Ansprüche, dadurch gekennzeichnet, daß Gemische von zwei oder mehreren der monoklonalen Antikörper verwendet werden.

FIG. I

# FIG. 2

FIG. 3